# EUROPEAN PATENT APPLICATION

(11) **EP 0 729 745 A1**
(43) Date of publication of application: **04.09.1996**
(21) Application number: 95301305.9
(22) Date of filing: 28.02.1995
(51) Int. Cl.: A61K 7/48, A61K 31/375, A61K 47/10

(54) **Vitamin C delivery system**

(71) Applicant: UNILEVER PLC, London EC4P 4BQ (GB); UNILEVER N.V., 3013 AL Rotterdam (NL)
(72) Inventor: Guerrero, Angel Augusto, Huntington, Connecticut 06484 (US); Vargas, Anthony, Monroe, Connecticut 06468 (US); Meyers, Alan Joel, Trumbull, Connecticut 06611 (US)
(74) Representative: Evans, Jacqueline Gail Victoria

(57) **Abstract**

An anhydrous cosmetic composition including
(i) from about 0.001 to about 50% by weight of ascorbic acid; and
(ii) from about 0.10 to about 99.9% by weight of a pharmaceutically acceptable carrier having the formula: 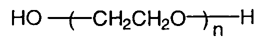 wherein n is an integer ranging from 2 to 50.

## Description

### BACKGROUND OF THE INVENTION

### Field of the Invention

The invention relates to a cosmetic product that can stably store ascorbic acid and then deliver same to the skin.

### The Related Art

Ascorbic acid, also known by its common name of Vitamin C, has long been recognized as an active substance benefiting skin appearance. Vitamin C reportedly increases the production of collagen in human skin tissue. Wrinkles and fine lines are thereby reduced. An overall healthier and younger-looking appearance results. Vitamin C has also found utility as an ultraviolet ray blocking or absorbing agent. Whitening or bleaching skin compositions have also employed Vitamin C utilizing its property of interference with the melanin formation process. There also is a belief that ascorbic acid interacts with the human immune system to reduce sensitivity to skin aggravating chemicals. Reduced levels of Vitamin C concentration on the skin have also been implicated with an increase in stress. From all of the foregoing perspectives, Vitamin C or ascorbic acid may provide significant benefit when topically applied.

Unfortunately, Vitamin C is a very unstable substance. Although it is readily soluble in water, rapid oxidation occurs in aqueous media. Solubility of ascorbic acid has been reported to be relatively poor in nonaqueous media, thereby preventing an anhydrous system from achieving any significant level of active concentration.

The art has sought to overcome the problem in a variety of ways. One approach is the preparation of ascorbic acid derivatives. These derivatives have greater stability than the parent compound and, through biotransformation or chemical hydrolysis, can at the point of use be converted to the parent acid. For instance, U.S. Patent 5,137,723 (Yamamoto et al) and U.S. Patent 5,078,989 (Ando et al) provide glycosylate and ester derivatives, respectively.

U.S. Patent 4,818,521 (Tamabuchi) describes under the background technology a so-called two-pack type cosmetic wherein Vitamin C powder and other ingredients are separately packaged in different containers with mixing just prior to use of the cosmetic. The mixing procedure and expensive packaging were said to be drawbacks of this system. The patent suggests stable oil-in-water type emulsions that are weakly acidic and wherein ascorbic acid has been premixed with a stabilizing oil.

Maintenance of pH below about 3.5 has also been suggested in U.S. Patent 5,140,043 (Darr et al) as a stabilization means for aqueous compositions of ascorbic acid.

Water compatible alcohols such as propylene glycol, polypropylene glycol and glycerol have been suggested as co-carriers alongside water to improve stability. An illustration of this approach can be found in U.S. Patent 4,983,382 (Wilmott and Znaiden). Therein a blend of water and water-miscible organic solvent are combined as a stabilizing system. At least about 40% of the organic solvent must be ethanol while the remainder may be selected from such alcohols as propylene glycol, glycerin, dipropylene glycol and polypropylene glycol.

U.S. Patent 4,372,874 (Modrovich) has reported incorporation of relatively large amounts of ascorbic acid in a polar water-miscible organic solvent such as dimethyl sulfoxide. Levels of water are kept below 0.5% through addition of a particulate desiccant to the carrier. Although highly polar systems such as dimethyl sulfoxide may be effective, this and related carriers are toxicologically questionable.

Accordingly, it is an object of the present invention to provide a delivery system for ascorbic acid in which the compound is storage stable.

Another object of the present invention is to provide a delivery system which assists the penetration of ascorbic acid into the human skin while avoiding irritation thereof.

Still another object of the present invention is to provide a system for delivering ascorbic acid that is sufficiently transparent so as to render the system aesthetically pleasing.

These and other objects of the present invention will become more readily apparent through the following summary, detailed discussion and Examples.

### SUMMARY OF THE INVENTION

An anhydrous cosmetic composition is provided that includes:
(i) from about 0.001 to about 50% by weight of ascorbic acid; and
(ii) from about 0.10 to about 99.9% by weight of a pharmaceutically acceptable carrier having the formula: 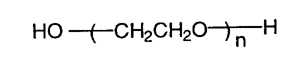 wherein n is an integer ranging from 2 to 50.

### DETAILED DESCRIPTION OF THE INVENTION

Now it has been discovered that ascorbic acid can be stably dissolved in an anhydrous composition containing a selected pharmaceutically acceptable carrier. The carrier of this invention is polyethylene glycol having the formula: 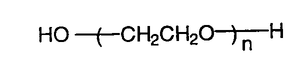 wherein n is an integer ranging from 2 to 50, preferably from 4 to 20, more preferably from 5 to 10.

Carbowax 400 and Carbowax 200 have been found to be the most useful polyethylene glycols.

Generally the carrier will be an ingredient present in highest amounts of the anhydrous composition. The amount can range from about 10 to about 99.9%, preferably from about 25 to about 90%, optimally between about 70 and 85% by weight.

Lower alcohols such as ethyl alcohol may be present to assist the carrier. However, the concentration of lower alcohol must be no higher than 40%, preferably ranging from 0.1 to about 25% by weight.

Another essential element of the present invention is, of course, ascorbic acid. The amount of ascorbic acid should range from about 0.001 to about 50%, preferably from about 0.01 to about 10%, optimally between about 3 and 6% by weight of the anhydrous composition.

Esters may also be incorporated into the anhydrous composition as emollients. Among esters that may be utilized are:
(1) Alkyl esters of fatty acids having 10 to 20 carbon atoms. Methyl, isopropyl, and butyl esters of fatty acids are useful herein. Examples include hexyl laurate, isohexyl laurate, isohexyl palmitate, isopropyl palmitate, decyl oleate, isodecyl oleate, hexadecyl stearate, decyl stearate, isopropyl isostearate, diisopropyl adipate, diisohexyl adipate, dihexyldecyl adipate, diisopropyl sebacate, lauryl lactate, myristyl lactate, and cetyl lactate. Particularly preferred are C₁₂-C₁₅ alcohol benzoate esters.
(2) Alkenyl esters of fatty acids having 10 to 20 carbon atoms. Examples thereof include oleyl myristate, oleyl stearate, and oleyl oleate.
(3) Ether-esters such as fatty acid esters of ethoxylated fatty alcohols.
(4) Polyhydric alcohol esters. Ethylene glycol mono and di-fatty acid esters, diethylene glycol mono- and di-fatty acid esters, polyethylene glycol (200-6000) mono-and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters.
(5) Wax esters such as beeswax, spermaceti, myristyl myristate, stearyl stearate.
(6) Sterols esters, of which cholesterol fatty acid esters are examples thereof.

Emulsifiers may also be present in the compositions of this invention. These emulsifiers may either be anionic, nonionic, cationic or amphoteric type. Nonionic emulsifiers are particularly preferred, especially polyoxyalkylene polyol fatty acid esters (which may also operate as ester emollients). Most preferred is polyethylene oxide (15) trimethylolpropane isostearate. Levels of emulsifier may range anywhere from about 0.1 to about 20%, preferably between about 1 and 5% by weight.

Silicone oils may also be included in the compositions of this invention. These oils may be either volatile or nonvolatile. The term "volatile" as used herein refers to those materials which have a measurable vapor pressure at ambient temperature. Volatile silicone oils are preferably chosen from cyclic or linear polydimethylsiloxanes containing from about 3 to about 9, preferably from about 4 to about 5, silicon atoms.

Linear volatile silicone materials generally have viscosities less than about 5 centistokes at 25°C while cyclic materials typically have viscosities of less than about 10 centistokes.

Examples of preferred volatile silicone oils useful herein include: Dow Corning 344, Dow Corning 345 and Dow Corning 200 (manufactured by Dow Corning Corp.); Silicone 7207 and Silicone 7158 (manufactured by the Union Carbide Corp.); SF 1202 (manufactured by General Electric); and SWS-03314 (manufactured by SWS Silicones, Inc.).

The nonvolatile silicone oils useful in compositions of this invention are exemplified by the polyalkyl siloxanes, polyalklyaryl siloxanes and polyether siloxane copolymers. The essentially nonvolatile polyalkyl siloxanes useful herein include, for example, polydimethyl siloxanes with viscosities of from about 5 to about 100,000 centistokes at 25°C. Among the preferred nonvolatile silicones useful in the present compositions are the polydimethyl siloxanes having viscosities from about 10 to about 400 centistokes at 25°C. Such polyalkyl siloxanes include the Viscasil series (sold by General Electric Company) and the Dow Corning 200 series (sold by Dow Corning Corporation). Polyalkylaryl siloxanes include poly(methylphenyl)siloxanes having viscosities of from about 15 to about 65 centistokes at 25°C. These are available, for example, as SF 1075 methylphenyl fluid (sold by General Electric Company) and 556 Cosmetic Grade Fluid (sold by Dow Corning Corporation). Useful polyether siloxane copolymers include, for example, a polyoxyalkylene ether copolymer having a viscosity of about 1200 to 1500 centistokes at 25°C. Such a fluid is available as SF-1066 organosilicone surfactant (sold by General Electric Company). Cetyl dimethicone copolyol and cetyl dimethicone are especially preferred because these materials also function as emulsifiers and emollients.

Among other skin benefit agents which may be present in the anhydrous compositions of this invention are fatty acids having from 10 to 20 carbon atoms. Suitable examples of the fatty acids include pelargonic, lauric, myristic, palmitic, stearic, isostearic, hydroxystearic, oleic, linoleic, ricinoleic, arachidic, behenic, and erucic acids. These materials may be present in amounts anywhere from about 0.1 to about 20% by weight of the composition, preferably between about 2 and 10% by weight of the anhydrous composition.

Acidifying agents may also be included in the anhydrous composition. These may be either organic or inorganic and ranging in amount from about 0.1 to about 20%, preferably between about 1 and 10%, optimally between about 2 and 6% by weight. The acids may include alginic acid, citric acid, malic acid, succinic acid, lactic acid, glycolic acid, tartaric acid, sorbic acid, phosphoric acid, acid phosphate salt, acid pyrophosate salt, bitartrate salt and metal acid citrate.

A preferred mode for utilization of the anhydrous cosmetic composition of the present invention is as one phase of a dual-phase cosmetic product. The second composition of the dual-phase product may be an aqueous composition containing an alkaline material for increasing pH when blended with the anhydrous composition. A multi-compartment dispenser is utilized for such product. A copending application having Attorney Docket No. 92-0310-EA describes this further development in the delivery of ascorbic acid.

The following examples will more fully illustrate the embodiments of this invention. All parts, percentages and proportions referred to herein and in the appended claims are by weight unless otherwise indicated.

### EXAMPLE 1

An anhydrous composition according to the present invention is set forth in the table below.

| Component | Wt. % |
|---|---|
| Carbowax 400 | 84.0 |
| Ascorbic Acid (Vitamin C) | 5.0 |
| Ethyl Alcohol | 5.0 |
| Anhydrous Citric Acid | 3.0 |
| Isostearic Acid | 3.0 |

### EXAMPLE 2

Another anhydrous composition according to the present invention is set forth in the table below.

| Component | Wt. % |
|---|---|
| Carbowax 200 | 82.0 |
| Ascorbic Acid (Vitamin C) | 5.0 |
| Ethyl Alcohol | 5.0 |
| Anhydrous Glycolic Acid | 3.0 |
| Isostearic Acid | 3.0 |
| Dimethicone | 2.0 |

### EXAMPLE 3

Another anhydrous composition according to the present invention is set forth in the table below.

| Component | Wt. % |
|---|---|
| Carbowax 400 | 83.0 |
| Ascorbic Acid (Vitamin C) | 5.0 |
| Glycerin | 5.0 |
| Anhydrous Lactic Acid | 3.0 |
| Linolenic Acid | 2.0 |
| Dimethicone Copolyol | 2.0 |

### EXAMPLE 4

Another anhydrous composition according to the present invention is set forth in the table below.

| Component | Wt. % |
|---|---|
| Carbowax 200 | 76.0 |
| Ascorbic Acid (Vitamin C) | 10.0 |
| Ethyl Alcohol | 5.0 |
| Anhydrous Malic Acid | 3.0 |
| Oleic Acid | 3.0 |
| Polyoxyethylene 15 Trimethylolpropane Isostearate | 3.0 |

### EXAMPLE 5

Another anhydrous composition according to the present invention is set forth in the table below.

| Component | Wt. % |
|---|---|
| Carbowax 400 | 80.9 |
| Isostearic Acid | 7.0 |
| Ascorbic Acid (Vitamin C) | 5.0 |
| Ethyl Alcohol | 5.0 |
| Polyoxyethylene 15 Trimethylolpropane Isostearate | 2.0 |
| Anhydrous Citric Acid | 0.1 |

### EXAMPLE 6

A variety of polyhydroxy compounds were evaluated as solvents for ascorbic acid. Table 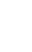 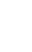 records solubility of ascorbic acid in these materials. The term "incompatible" reflects a material that had visible signs of chemical reaction with ascorbic acid. Those deemed to be insoluble did not solubilize enough ascorbic acid to produce a stable two-phase product. From the table it is evident that only polyethylene glycol provided suitable solubility and compatibility.

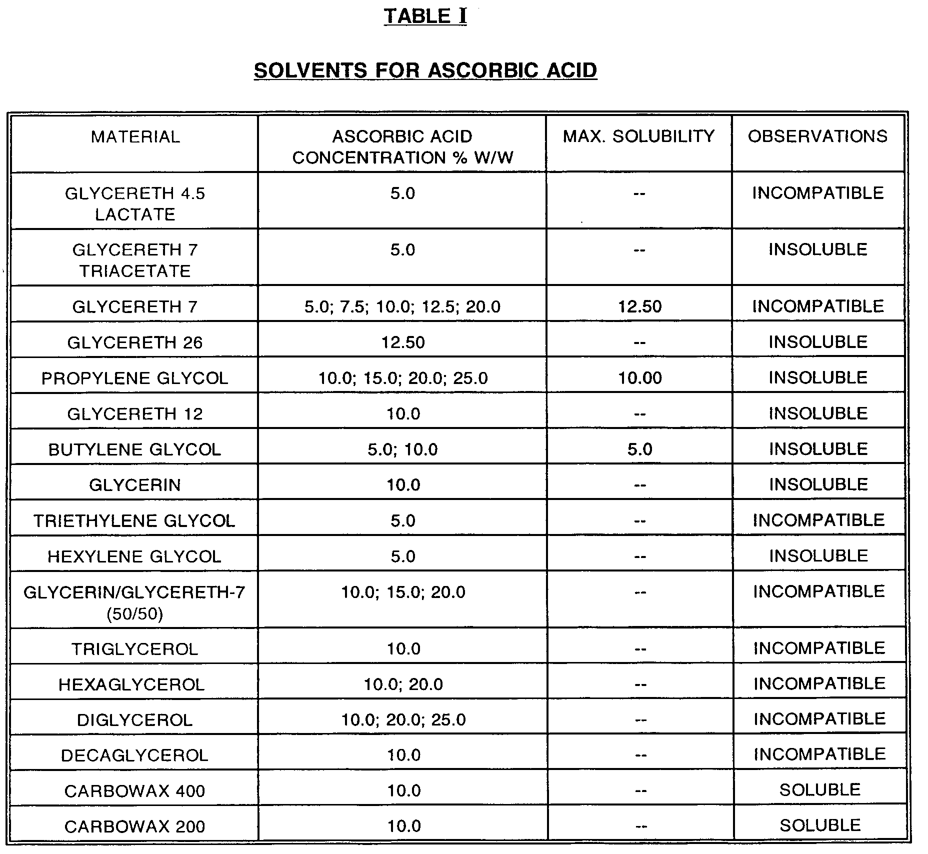

The foregoing description and examples illustrate selected embodiments of the present invention and in light thereof variations and modifications will be suggested to one skilled in the art, all of which are in the spirit and purview of this invention.

## Claims

1. An anhydrous cosmetic composition comprising:
(i) from about 0.001 to about 50% by weight of ascorbic acid; and
(ii) from about 0.10 to about 99.9% by weight of a pharmaceutically acceptable carrier having the formula: 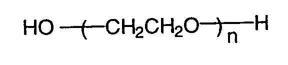 wherein n is an integer ranging from 2 to 50;

2. The cosmetic composition according to claim 1 wherein the ascorbic acid is present in an amount from about 0.01 to about 10% by weight of the cosmetic composition.

3. The cosmetic composition according to claim 1 further comprising an acidifying agent present in an amount from about 0.1 to about 20% by weight of the composition.

4. The cosmetic composition according to claim 1 wherein n is an integer from 5 to 10.

5. The cosmetic composition according to claim 1 further comprising an emulsifier present in an amount from about 0.5 to about 20% by weight.

6. The cosmetic composition according to claim 5 wherein the emulsifier is polyoxyalkylene polyol fatty acid ester.

7. The emulsifier according to claim 6 which is polyethylene oxide (15) trimethylolpropane isostearate.
